# EUROPÄISCHE PATENTSCHRIFT

(11) **EP 1 827 415 B1**
(45) Veröffentlichungstag und Bekanntmachung des Hinweises auf die Patenterteilung: **20.01.2010**
(21) Anmeldenummer: 05814551.7
(22) Anmeldetag: 30.11.2005
(51) Int. Cl.: A61K 31/215, A61P 3/06, A61P 3/10, A61P 43/00

(54) **CYCLOPROPANSÄUREDERIVATEN ZUR SENKUNG DES LIPIDSPIEGELS**
CYCLOPROPANE ACID DERIVATIVES FOR THE REDUCTION OF LIPID LEVELS
DERIVES D'ACIDE CYCLOPROPANIQUE ABAISSANT LE TAUX LIPIDIQUE

(30) Priorität: 14.12.2004 DE 102004060041; 19.08.2005 DE 102005039245
(43) Veröffentlichungstag der Anmeldung: 05.09.2007
(73) Patentinhaber: Sanofi-Aventis Deutschland GmbH, 65929 Frankfurt am Main (DE)
(72) Erfinder: KADEREIT, Dieter, 63071 Offenbach (DE); STENGELIN, Siegfried, 65817 Eppstein (DE); HEUER, Hubert, 55270 Schwabenheim (DE); BRUMMERHOP, Harm, 60389 Frankfurt (DE)
(86) Internationale Anmeldenummer: PCT/EP2005/012763
(87) Internationale Veröffentlichungsnummer: WO 2006/063681

(56) Entgegenhaltungen:
- WO-A-02/066469
- WO-A-03/053352
- WO-A-03/053915
- GRANDJEAN, D; PALE, P; CHUCHE, J: "Enzymatic hydrolysis of cyclopropanes. Total synthesis of optically pure dictyopterenes A and C'." TETRAHEDRON, Bd. 47, Nr. 7, 1991, Seiten 1215-1230, XP002371232

## Beschreibung

Die Erfindung betrifft die Verwendung von substituierten Cyclopropansäurederivaten sowie derer physiologisch verträglichen Salze zur Herstellung von Medikamenten zur Behandlung des Metabolischen Syndroms.

In WO2002/066469 sind substituierte Cyclopropansäurederivate als Dopamin Rezeptor Liganden beschrieben.
Der Erfindung lag die Aufgabe zugrunde, Verbindungen zur Verfügung zu stellen, die zur Behandlung des Metabolischen Syndroms verwendet werden können und die insbesonders eine therapeutisch verwertbare lipidsenkende Wirkung entfalten. Weiter bevorzugt sollten sie zur Behandlung der Diabetischen Dyslipidämie geeignet sein.Weiter bevorzugt sollte eine Senkung der freien Fettsäuren (FFA), von Glycerol und der Triglyceride im Plasma erreicht werden.

Die Erfindung betrifft daher die Verwendung der Verbindungen der Formel I, 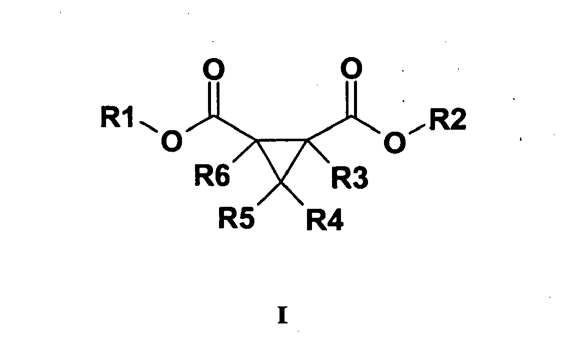 worin bedeuten
- R1: H;
- R2: (C₁-C₁₀)-Alkyl, (C₂-C₁₀)-Alkenyl, (C₂-C₁₀)-Alkinyl, wobei die Alkyl-, Alkenyl- und Alkinylreste ein oder mehrfach substituiert sein können mit F, Cl, Br, CF₃, NO₂, CN, COOH, COO(C₁-C₆)Alkyl, (C₃-C₇)-Cycloalkyl, (C₁-C₁₀)-Alkyl, (C₂-C₆)-Alkenyl, (C₂-C₆)-Alkinyl, O-(C₁-C₆)-Alk-yl, O-CO-(C₁-C₆)-Alkyl, O-CO-(C₁-C₆)-Aryl;
- R3, R4, R5, R6: unabhängig voneinander H, F, (C₁-C₄)-Alkyl; sowie derer physiologisch verträglichen Salze.

Die Erfindung bezieht sich auf die Verwendung der Verbindungen der Formel I, in Form ihrer Racemate, racemischen Mischungen und reinen Enantiomere sowie auf ihre Diastereomere und Mischungen davon.

Können Reste oder Substituenten mehrfach in den Verbindungen der Formel I auftreten, so können sie alle unabhängig voneinander die angegebenen Bedeutungen haben und gleich oder verschieden sein.

Unter einem Alkylrest wird eine geradkettige oder verzweigte Kohlenwasserstoffkette mit einem oder mehreren Kohlenstoffen verstanden, wie z.B. Methyl, Ethyl, Propyl, Butyl, Hexyl, Isopropyl, Isobutyl, Neopentyl, tert.-Butyl, Hexyl.
Die Alkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, Aryl, Heterozyklyl, O-(C₁-C₆)-Alkyl, O-COO-(C₁-C₆)-Alkyl, O-CO-(C₁-C₆)-Alkyl, O-CO-(C₁-C₆)-Aryl, O-CO-(C₁-C₆)-Heterozyklyl, PO₃H₂, P(O)(OAlkyl)₂, (C₁-C₆)-Alkylen-P(O)(OAlkyl)2, O-P(O)(OH)₂, O-P(O)(OAlkyl)2, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N[((C₁-C₆)-Alkyl)(CH₂)ₙ-Aryl], SO₂-N[((C₁-C₆)-Alkyl)(CH₂)ₙHeterozyklyl], SO₂-N((CH₂)ₙ-Aryl)₂,, SO₂-N((CH₂)ₙ-(Heterozyklyl))₂, wobei n = 0 - 6 sein kann und der Aryl- oder Heterozyklyl- Rest bis zu dreifach mit F, Cl, Br, OH, CF₃, SF₅, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Akyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.

Unter einem Alkenylrest wird eine geradkettige oder verzweigte Kohlenwasserstoffkette mit zwei oder mehreren Kohlenstoffen sowie einer oder mehreren Doppelbindungen verstanden, wie z.B. Vinyl, Allyl, Pentenyl.

Die Alkenylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkinyl, Aryl, Heterozyklyl, O-(C₁-C₆)-Alkyl O-CO-(C₁-C₆)-Alkyl, O-CO-(C₁-C₆)-Aryl, O-CO-(C₁-C₆)-Heterozyklyl,;

PO₃H₂, P(O)(OAlkyl)₂, (C₁-C₆)-Alkylen-P(O)(OAlkyl)₂, O-P(O)(OH)₂, O-P(O)(OAlkyl)₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl; SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO₂-N((CH₂)ₙ-Aryl)₂,, SO₂-N((CH₂)ₙ-(Heterozyklyl)₂ wobei n = 0 - 6 sein kann und der Arylrest oder heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.

Unter einem Alkinylrest wird eine geradkettige oder verzweigte Kohlenwasserstoffkette mit zwei oder mehreren Kohlenstoffen sowie einer oder mehreren Dreifachbindungen verstanden, wie z.B. Ethinyl, Propinyl, Hexinyl.
Die Alkenylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₁-C₁₀)-Alkyl, O-(C₁-C₆)-Alkyl O-CO-(C₁-C₆)-Alkyl, O-CO-(C₁-C₆)-Aryl, O-CO-(C₁-C₆)-Heterozyklyl;
PO₃H₂, P(O)(OAlkyl)₂, (C₁-C₆)-Alkylen-P(O)(OAlkyl)₂, O-P(O)(OH)₂, O-P(O)(OAlkyl)₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C)-C₆)-Alkyl)(CH₂)ₙ-Heterozyklyl, SO₂-N((CH₂)ₙ-Aryl)₂,, SO₂-N((CH₂)ₙ-(Heterozyklyl)₂ wobei n = 0 - 6 sein kann und der Arylrest oder Heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann;
C(NH)(NH₂), NH, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C6)₋Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl,
N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Akyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.
Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.
Die Arylreste können ein oder mehrfach mit geeigneten Gruppen substituiert sein, wie z.B.: F, Cl, Br, I, CF₃, NO₂, SF₅, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl,;
PO₃H₂, P(O)(OAlkyl)₂, (C₁-C₆)-Alkylen-P(O)(OAlkyl)₂, O-P(O)(OH)₂, O-P(O)(OAlkyl)₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Akyl)(CH₂)ₙ-Heterozyklyl, SO₂-N((CH₂)ₙ-Aryl)₂, SO₂-N((CH₂)ₙ-(Heterozyklyl)₂ wobei n = 0 - 6 sein kann und der Arylrest oder Heterozyklische Rest bis zu zweifach mit F, Cl, Br, OH, CF₃, NO₂, SF₅, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C6)-Alkyl]-CO-NH-Heterozyklyl, N[(C₁-C₆)-Alkyl]-CO-N((C₁-C₆)-Akyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.
Die Cycloalkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl,;
PO₃H₂, P(O)(OAlkyl)₂, (C₁-C₆)-Alkylen-P(O)(OAlkyl)₂, O-P(O)(OH)₂, O-P(O)(OAlkyl)₂, SO₃H, SO₂-NH2, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N((C₁-C₆)-Alkyl)(CH₂)ₙ-Aryl, SO₂-N((C₁-C₆)-Alkyl)(CH₂)ₙ-Heterozyklyl, SO₂-N((CH₂)ₙ-Aryl)₂,, SO₂-N((CH₂)ₙ-(Heterozyklyl)₂ wobei n = 0 - 6 sein kann und der Arylrest oder Heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C₆)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Aryl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.

Unter Heterozyklus, Heterozyklyl bzw. heterozyklischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der heterozyklische Rest mit Benzolkernen kondensiert ist.

Geeignete Heterozyklyl- bzw. "heterozyklische Reste" sind Acridinyl, Benzimidazolyl, Benzofuryl, Benzothienyl, Benzothiophenyl, Benzoxazolyl, Benzthiazolyl, Benztriazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazol, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl, Aziridinyl, Azetininyl, Azepanyl, Azocanyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-Pyridyl.

Umfasst sind weiterhin ein oder mehrfach benzoannelierte Derivate dieser Heterozyklen.

Die heterozyklischen Ringe bzw. Heterozyklische Reste können ein oder mehrfach mit geeigneten Gruppen substituiert sein, wie z.B: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterozyklyl;
PO₃H₂, P(O)(OAlkyl)₂, (C₁-C₆)-Alkylen-P(O)(OAlkyl)₂ O-P(O)(OH)₂, O-P(O)(OAlkyl)₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterozyklyl, SO-(C₁-C₆)-Alkyl, SO-(CH₂)ₙ-Aryl, SO-(CH₂)ₙ-Heterozyklyl, SO₂-(C₁-C₆)-Alkyl, SO₂-(CH₂)ₙ-Aryl, SO₂-(CH₂)ₙ-Heterozyklyl, SO₂-NH(CH₂)ₙ-Aryl, SO₂-NH(CH₂)ₙ-Heterozyklyl, SO₂-N(C₁-C₆)-Alkyl)(CH₂)ₙ-Aryl, SO₂-N(C₁-C₆)-Alkyl)(CH₂)ₙ-Heterozyklyl, SO₂-N((CH₂)ₙ-Aryl)₂,, SO₂-N((CH₂)ₙ-(Heterozyklyl)₂ wobei n = 0 - 6 sein kann und der Arylrest oder heterozyklische Rest bis zu zweifach mit F, Cl, Br, OH, CF₃, SF₅, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterozyklyl, NH-COO-Aryl, NH-COO-Heterozyklyl, NH-CO-NH-(C₁-C₆)-Alkyl), NH-CO-NH-Aryl, NH-CO-NH-Heterozyklyl, N[(C₁-C₆)-Alkyl]-CO-(C₁-C₆)-Alkyl, N[(C₁-C₆)-Alkyl]-COO-(C₁-C₆)-Alkyl, N[(C₁-C₆)-Alkyl]-CO-Aryl, N[(C₁-C₆)-Alkyl]-CO-Heterozyklyl, N[(C₁-C₆)-Alkyl]-COO-Aryl, N[(C₁-C₆)-Alkyl]-COO-Heterozyklyl, N[(C₁-C₆)-Alkyl]-CO-NH-(C₁-C₆)-Alkyl), N[(C₁-C₆)-Alkyl]-CO-NH-Aryl, N[(C₁-C₆)-Alkyl]-CO-NH-Heterozyklyl, N[(C₁-C₆)-Alkyl]-CO-N((C₁-C₆)-Alkyl)₂, N[(C₁-C₆)-Alkyl]-CO-N((C₁-C₆)-Alkyl)-Aryl, N[(C₁-C₆)-Alkyl]-CO-N((C₁-C₆)-Alkyl)-Heterozyklyl, N[(C₁-C₆)-Alkyl]-CO-N(Aryl)₂, N[(C₁-C6)-Alkyl]-CO-N(Heterozyklyl)₂, N(Aryl)-CO-(C₁-C₆)-Alkyl, N(Heterozyklyl)-CO-(C₁-C₆)-Alkyl, N(Aryl)-COO-(C₁-C₆)-Alkyl, N(Heterozyklyl)-COO-(C₁-C₆)-Alkyl, N(Aryl)-CO-Myl, N(Heterozyklyl)-CO-Aryl, N(Aryl)-COO-Aryl, N(Heterozyklyl)-COO-Aryl, N(Aryl)-CO-NH-(C₁-C₆)-Alkyl, N(Heterozyklyl)-CO-NH-(C₁-C₆)-Alkyl, N(Aryl)-CO-NH-Aryl, N(Heterozyklyl)-CO-NH-Aryl, N(Aryl)-CO-N((C₁-C₆)-Alkyl)₂, N(Heterozyklyl)-CO-N((C₁-C₆)-Alkyl)₂, N(Aryl)-CO-N[(C₁-C₆)-Alkyl]-Aryl, N(Heterozyklyl)-CO-N[(C₁-C₆)-Alkyl]-Aryl, N(Aryl)-CO-N(Aryl)₂, N(Heterozyklyl)-CO-N(Aryl)₂, Aryl, O-(CH₂)ₙ-Aryl, O-(CH₂)ₙ-Heterozyklyl, wobei n = 0 - 6 sein kann, wobei der Aryl- oder Heterozyklyl- Rest ein bis 3-fach substituiert sein kann mit F, Cl, Br, I, OH, CF₃, NO₂, CN, OCF₃, SF₅, O-(C₁-C₆)-Alkyl, (C₁-C₆)-Alkyl, NH₂, NH(C₁-C₆)-Alkyl, N((C₁-C₆)-Alkyl)₂, SF₅, SO₂-CH₃, COOH, COO-(C₁-C₆)-Alkyl, CONH₂.

Pharmazeutisch verträgliche Salze sind aufgrund ihrer höheren Wasserlöslichkeit gegenüber den Ausgangs- bzw. Basisverbindungen besonderes geeignet für medizinische Anwendungen. Diese Salze müssen ein pharmazeutisch verträgliches Anion oder Kation aufweisen. Geeignete pharmazeutisch verträgliche Säureadditionssalze der Verbindungen der Formel I sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isäthion-, Milch-, Lactobion-, Malein-, Apfel-, Methansulfon-, Bernstein-, p-Toluolsulfon-, Wein- und Trifluoressigsäure ferner L-Ascorbinsäure, Salizylsäure, 1,2-Benzisothiazol-3(2H)-on und 6-Methyl-1,2,3-oxathiazin-4(3H)-on-2,2-dioxid. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die Verbindungen der Formel I können auch in verschiedenen polymorphen Formen vorliegen, z.B. als amorphe und kristalline polymorphe Formen. Alle polymorphen Formen der Verbindungen der Formel I gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze und Solvate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen-für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Benzothiazepin-Ions. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharrriaceutical Research, 2(6): 318 (1986) beschrieben, durch Elektrotransport oder Iontophorese freigesetzt werden.

Die nachfolgend aufgeführten Beispiele dienen zur Erläuterung der Erfindung, ohne diese jedoch einzuschränken.

| Beispiel | Formel | Name | Salz |
|---|---|---|---|
| 1 | 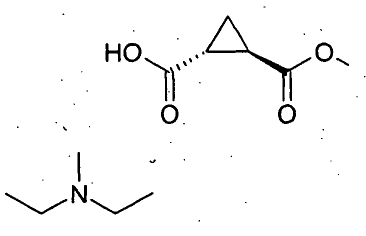 | 1R-Methoxycarbonyl-2R-hydroxycarbonyl-cyclopropan | Diethylmethylamin |
| 2 | 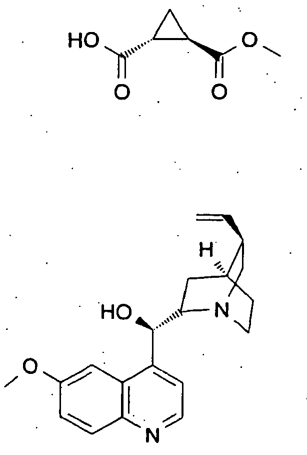 | 1R-Methoxycarbonyl-2R-hydroxycarbonyl-cyclopropan | Chinidin |
| 3 | 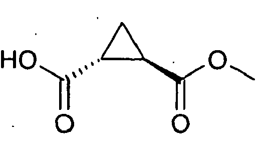 | 1R-Methoxycarbonyl-2R-hydroxycarbonyl-cyclopropan | |
| 4 | 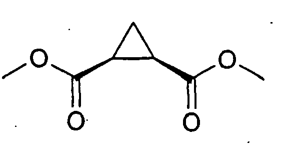 | cis-1,2-Cyclopropandicarbonsäure-dimethylester | |
| 5 | 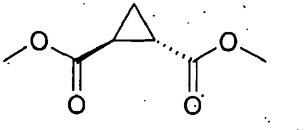 | trans-1,2-Cyclopropandicarbonsäure-dimethylester | |
| 6 | 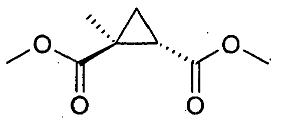 | 1-Methyl-trans-cyclopropan-1,2-dicarbonsäure-dimethylester | |
| 7 | 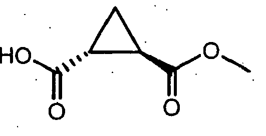 | 1RS-Methoxycarbonyl-2RS-hydroxycarbonyl-cyclopropan | |
| 8 | 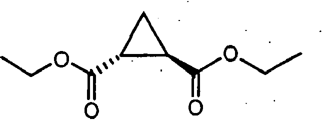 | trans-1,2-Cyclopropandicarbonsäure-diethylester | |
| 9 | 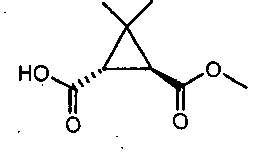 | 1RS-Methoxycarbonyl-2RS-hydroxycarbonyl-3,3-dimethyl-cyclopropan | |
| 10 | 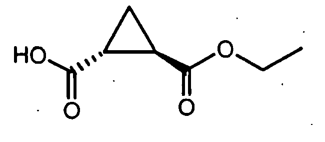 | 1RS-Ethoxycarbonyl-2RS-hydroxycarbonyl-cyclopropan | |
| 11 | 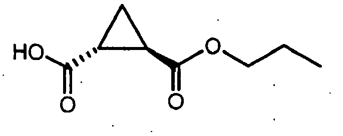 | 1RS-Propoxycarbonyl-2RS-hydroxycarbonyl-cyclopropan | |
| 12 | 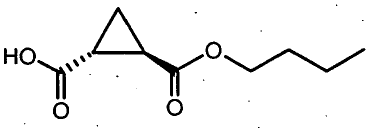 | 1RS-Butoxycarbonyl-2RS-hydroxycarbonyl-cyclopropan | |
| 13 | 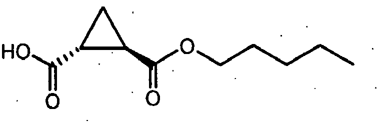 | 1RS-Pentoxycarbonyl-2RS-hydroxycarbonyl-cyclopropan | |
| 14 | 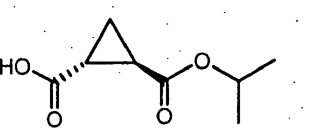 | 1RS-iso-Propoxycarbonyl-2RS-hydroxycarbonyl-cyclopropan | |
| 15 | 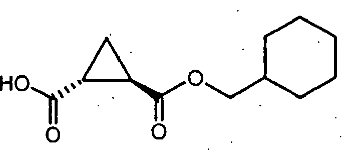 | 1 RS-Cyclohexylmethyloxycarbon yl-2RS-hydroxycarbonyl-cyclopropan | |
| 16 | 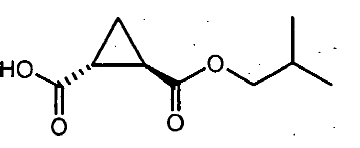 | 1 RS-isobutoxycarbonyl-2RS-hydroxycarbonyl-cyclopropan | |
| 17 | 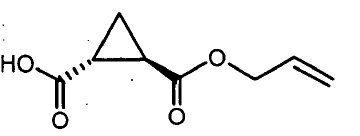 | 1 RS-Allyloxycarbonyl-2RS-hydroxycarbonyl-cyclopropan | |
| 18 | 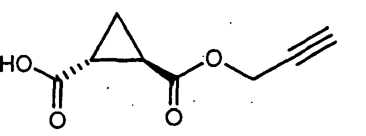 | 1RS-Propargyloxycarbonyl-2RS-hydroxycarbonyl-cyclopropan | |
| 19 | 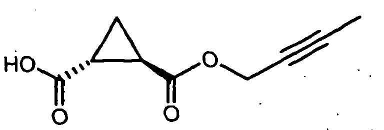 | 1RS-Butinyloxycarbonyl-2RS-hydroxycarbonyl-cyclopropan | |
| 20 | 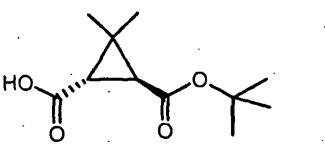 | 1 RS-tertButoxycarbonyl-2RS-hydroxycarbonyl-3,3-dimethyl-cyclopropan | |
| 21 | 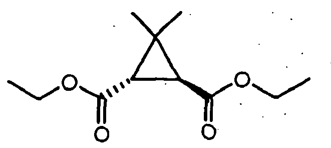 | 3,3-Dimethyl-trans-1,2-Cyclopropandicarbonsäure-diethylester | |

Die Verbindungen der Formel I eignen sich zur Behandlung des Metabolischen Syndroms (siehe Datamonitor 11/2002, Kapitel 2, Seiten 19- 32), zur Prädiabetes Behandlung und zur Prophylaxe von Diabetes Typ 2. Sie eignen sich insbesonders zur Behandlung der Diabetischen Dyslipidämie. Die diabetische Dyslipidämie manifestiert sich in einer Erhöhung der Plasmatriglyzeride, einer Verminderung des HDL-Cholesterins sowie oft in erhöhten LDL-Spiegeln. Durch das gehäufte Auftreten kleiner, dichter LDL-Cholesterinpartikel mit hoher atherogener Potenz ist die Diabetische Dyslipidämie ein starker kardiovaskulärer Risikofaktor.

Die Verbindungen der Formel I können beispielsweise in folgenden Zubereitungen formuliert werden:

### Beispiel A

Gelatineweichkapseln, enthaltend 100 mg Wirkstoff pro Kapsel:

| | pro Kapsel |
|---|---|
| Wirkstoff | 100 mg |
| aus Kokosfett fraktioniertes | |
| Triclycerid-Gemisch | 400 mg |
| Kapselinhalt | 500 mg |

### Beispiel B

Emulsion, enthaltend 60 mg Wirkstoff pro 5 ml:

| | pro 100 ml Emulsion |
|---|---|
| Wirkstoff | 1,2 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

### Beispiel C

Rektale Arzneiform, enthaltend 40 mg Wirkstoff pro Suppositorium:

| | pro Suppositorium |
|---|---|
| Wirkstoff | 40 mg |
| Suppositoriengrundmasse | ad 2 g |

### Beispiel D

Tabletten, enthaltend 40 mg Wirkstoff pro Tablette:

| | pro Tablette |
|---|---|
| Lactose | 600 mg |
| Maisstärke | 300 mg |
| lösliche Stärke | 20 mg |
| Magnesiumstearat | 40 mg |
| | 1000 mg |

### Beispiel E

Dragees, enthaltend 50 mg Wirkstoff pro Dragees:

| | pro Dragee |
|---|---|
| Wirkstoff | 50 mg |
| Maisstärke | 100 mg |
| Lactose | 60 mg |
| sec. Calciumphosphat | 30 mg |
| lösliche Stärke | 5 kg |
| Magnesiumstearat | 10 mg |
| kolloidale Kieselsäure | 5 mg |
| | 260 mg |

### Beispiel F

Für die Herstellung des Inhalts von Hartgelatinekapseln eignen sich die folgenden Rezepturen:

| | |
|---|---|
| a) Wirkstoff | 100 mg |
| Maisstärke | 300 mg |
| | 400 mg |
| b) Wirkstoff | 140 mg |
| Milchzucker | 180 mg |
| Maisstärke | 180 mg |
| | 500 mg |

### Beispiel G

Tropfen können nach folgender Rezeptur hergestellt werden (100 mg Wirkstoff in 1 ml = 20 Tropfen):

| | |
|---|---|
| Wirkstoff | 10 g |
| Benzoesäuremethylester | 0,07 g |
| Benzoesäureethylester | 0,03 g |
| Ethanol 96 %ig | 5 ml |
| entmineralisiertes Wasser | ad 100 ml |

### Die Wirksamkeit der Verbindungen der Formel I wurde wie folgt getestet:

### Biologisches Prüfmodell:

### Die Prüfung der Wirkung erfolgte

Am Ganztier (Maus, Ratte, Hamster oder Hund) wird nach einer Fastenperiode (z.B. von ca. 16 Stunden) die Substanz appliziert (z.B. p.o., iv., i.p., s.c.) und mit oder ohne zusätzliche Stimulation der endogenen Lipolyse (z.B. durch eine Bolusinjektion von 2 mg/kg i.p. Isoprenaline) die Wirkung der Testsubstanz auf die Lipolyse anhand der freigesetzten freien Fettsäuren (FFA), Glycerol und Triglyceride bestimmt, indem z.B. 15 min, 30, 60, 120 usw. Minuten nach p.o. Applikation eine Blutprobe (z.B. durch retroorbitale Blutentnahme) gewonnen wird und nach Standard klinisch-chemischen Methoden (z.B. L.Thomas: Labor und Diagnose, 2. Auflage, Medizinische Verlagsgesellschaft, Marburg/L. 1984; ISBN 3-921320-10-9)) analysiert wird. Die Hemmung der Lipolyse durch die Inhibitoren wird im Vergleich zur Lipolyserate an entsprechend behandelten Kontrolltieren ausgewertet.
Beispiel 5 wurde in Dosen von 0,3 bis 30 mg/kg po verabreicht, wodurch die Lipolyse deutlich abgesenkt wurde, wie an der Reduktion von freien Fettsäuren (FFA), Glycerol und Triglyceriden gezeigt werden konnte.

| FFA | | |
|---|---|---|
| Zeitpunkt | Kontrolle n=15 | 3mg/kg n=6 |
| 0 | 0.47 | 0.47 |
| 15 | 0.60 | 0.17 |
| 30 | 0.62 | 0.13 |
| 60 | 0.62 | 0.11 |
| 120 | 0.69 | 0.25 |

| Glycerin | | |
|---|---|---|
| Zeitpunkt | Kontrolle n=15 | 3mg/kg n=6 |
| 0 | 89 | 87 |
| 15 | 98 | 39 |
| 30 | 94 | 33 |
| 60 | 96 | 28 |
| 120 | 130 | 57 |

| Triglyceride | | |
|---|---|---|
| Zeitpunkt | Kontrolle n=14 | 3mg/kg n=6 |
| 0 | 0.36 | 0.45 |
| 15 | 0.40 | 0.45 |
| 30 | 0.38 | 0.26 |
| 60 | 0.37 | 0.15 |
| 120 | 0.37 | 0.13 |

"n" steht für die Anzahl der Tiere. Es wurden Wistar Ratten getestet.

Die Wirksamkeit der Verbindungen der Formel I wurde auch in folgenden Assays getestet:
In vitro funktionale Assays mit rekombinanten Zellen
Funktionsüberprüfende Assays wurden mittels der FLIPR-Technik ("Fluorescence Imaging Plate Reader", Molecular Devices Corp.) durchgeführt. Hierzu wurden agonist-induzierte Änderungen der intrazellulären Konzentration von Ca²⁺ in rekombinanten HEK293 Zellen bestimmt, die sowohl den GPCR HM74A (Niacin-Receptor), als auch das hybride G-protein Gα6qi4myr (siehe z.B. Patentanmeldung DE10033353) exprimieren.
Für die Untersuchungen wurden Zellen in 96-well-Mikrotiterplatten (60000 Zellen/well) ausgesät und übernacht wachsengelassen. Das Medium wurde entfernt und die Zellen in Puffer inkubiert, der den Fluoreszenzfarbstoff Fluo-4 enthielt. Nach dieser Beladung mit Farbstoff wurden die Zellen gewaschen, Testsubstanz zugegeben und Änderungen in der intrazellulären Ca²⁺-Konzentration im FLIPR-Gerät gemessen. Ergebnisse wurden als prozentuale Änderung relativ zur Kontrolle dargestellt (0%: keine Testsubstanz addiert; 100%: 1 µM Referenzagonist Niacin addiert), zur Berechnung von.Dosis/Wirkungskurven verwendet und EC50-Werte bestimmt.

**Tabelle 2: Biologische Aktivität**

| Beispiel Nr. | EC50 (HM74a) µM |
|---|---|
| 1 | 0.07 |
| 2 | 0.13 |
| 3 | 0.19 |
| 4 | > 30 |
| 5 | > 30 |
| 6 | > 30 |
| 7 | 0.63 |
| 8 | > 30 |
| 9 | > 30 |
| 10 | 1.48 |
| 11 | 2.16 |
| 12 | 0.67 |
| 13 | 1.61 |
| 14 | > 30 |
| 15 | > 30 |
| 16 | > 30 |
| 17 | 2.83 |
| 18 | 1.51 |
| 19 | 1.63 |
| 20 | > 30 |
| 21 | > 30 |

Die Verbindungen der Beispiele 1, 4, 5, 6, 8 und 21 wurden von Chemikalienanbietern wie Fluka^{®}, Aldrich^{®} oder Acros^{®} gekauft. Die Verbindungen der Beispiele 2 und 3 sind aus WO 2002/066469 bekannt.

Synthese von (1RS)-Pentoxycarbonyl-(2RS)-hydroxycarbonyl-cyclopropan (Beispiel 13):

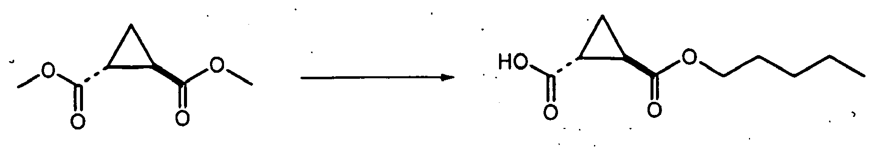

250 mg (1.58 mmol) trans-Cyclopropan-1,2-dicarbonsäuredimethylester werden in 5 mL 1-Pentanol gelöst und mit 0.36 mL einer 5-molaren, wässrigen NaOH-Lösung versetzt. Das Reaktionsgemisch wird 5 Tage bei 25 °C gerührt. Anschließend werden 50 mL Wasser zugegeben und das Reaktionsgemisch wird 2 mal mit jeweils 30 mL Essigsäureethylester extrahiert. Die organische Phase wird verworfen, und die wässrige Phase wird mit 2-molarer Salzsäure auf pH = 2 eingestellt und 3 mal mit Essigsäureethylester extrahiert. Die vereinigte organische Phase wird über MgSO₄ getrocknet und am Rotationsverdampfer eingeengt.
Man erhält 220 mg (1.10 mmol) 1RS-Pentoxycarbonyl-2RS-hydroxycarbonyl-cyclopropan als farbloses Öl.

Die Beispiele 7, 9, 10, 11, 12, 14, 15, 16, 17, 18, 19 und 20 wurden analog dieser allgemeinen Arbeitsvorschrift ausgehend vom entsprechenden Dimethylester oder Diethylester synthetisiert.

## Patentansprüche

1. Verwendung der Verbindungen der Formel I, 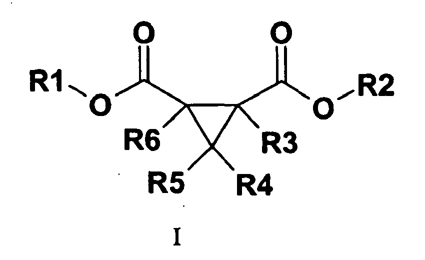 worin bedeuten
R1 H;
R2 (C₁-C₁₀)-Alkyl, (C₂-C₁₀)-Alkenyl, (C₂-C₁₀)-Alkinyl, wobei die Alkyl-, Alkenyl- und Alkinylreste ein oder mehrfach substituiert sein können mit F, Cl, Br, CF₃, NO₂, CN, COOH, COO(C₁-C₆)Alkyl, (C₃-C₇)-Cycloalkyl, (C₁-C₁₀)-Alkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl;
R3, R4, R5, R6 unabhängig voneinander H, F, (C₁-C₄)-Alkyl; sowie derer physiologisch verträglichen Salze zur Herstellung eines Medikaments zur Behandlung des Metabolischen Syndroms.

2. Verwendung der Verbindungen der Formel I, gemäß Anspruch 1, sowie derer physiologisch verträglichen Salze zur Herstellung eines Medikaments zur Lipidsenkung im Plasma.

3. Verwendung der Verbindungen der Formel I, gemäß Anspruch 1, sowie derer physiologisch verträglichen Salze zur Herstellung eines Medikaments zur Senkung der freien Fettsäuren (FFA) im Plasma.

4. Verwendung der Verbindungen der Formel I, gemäß Anspruch 1, sowie derer physiologisch verträglichen Salze zur Herstellung eines Medikaments zur Senkung von Glycerol im Plasma.

5. Verwendung der Verbindungen der Formel I, gemäß Anspruch 1, sowie derer physiologisch verträglichen Salze zur Herstellung eines Medikaments zur Senkung der Triglyceride im Plasma.

6. Verwendung der Verbindungen der Formel I, gemäß Anspruch 1, sowie derer physiologisch verträglichen Salze zur Herstellung eines Medikaments zur Prophylaxe von Diabetes Typ 2.

7. Verwendung der Verbindungen der Formel I, gemäß Anspruch 1, sowie derer physiologisch verträglichen Salze zur Herstellung eines Medikaments zur Behandlung der Diabetischen Dyslipidämie.

## Claims

1. The use of the compounds of the formula I, 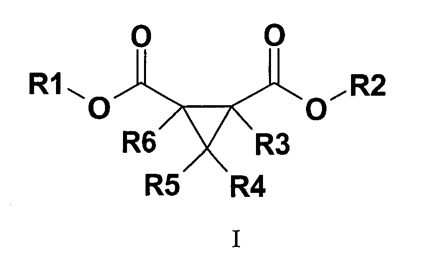 in which the meanings are
R1 H;
R2 (C₁-C₁₀)-alkyl, (C₂-C₁₀)-alkenyl, (C₂-C₁₀)-alkynyl, where the alkyl, alkenyl and alkynyl radicals may be substituted one or more times by F, Cl, Br, CF₃, NO₂, CN, COOH, COO(C₁-C₆)alkyl, (C₃-C₇)-cycloalkyl, (C₁-C₁₀)-alkyl, (C₂-C₆)-alkenyl, (C₂-C₆)-alkynyl, O-(C₁-C₆)-alkyl, O-CO-(C₁-C₆)-alkyl, O-CO-(C₁-C₆)-aryl;
R3, R4, R5, R6 independently of one another H, F, (C₁-C₄)-alkyl;
and of the physiologically tolerated salts thereof, for producing a medicament for the treatment of the metabolic syndrome.

2. The use of the compounds of the formula I as claimed in claim 1 and of the physiologically tolerated salts thereof for producing a medicament for reducing plasma lipids.

3. The use of the compounds of the formula I as claimed in claim 1 and of the physiologically tolerated salts thereof for producing a medicament for reducing plasma free fatty acids (FFA).

4. The use of the compounds of the formula I as claimed in claim 1 and of the physiologically tolerated salts thereof for producing a medicament for reducing plasma glycerol.

5. The use of the compounds of the formula I as claimed in claim 1 and of the physiologically tolerated salts thereof for producing a medicament for reducing plasma triglycerides.

6. The use of the compounds of the formula I as claimed in claim 1 and of the physiologically tolerated salts thereof for producing a medicament for the prophylaxis of type 2 diabetes.

7. The use of the compounds of the formula I as claimed in claim 1 and of the physiologically tolerated salts thereof for producing a medicament for the treatment of diabetic dyslipidemia.

## Revendications

1. Utilisation de composés de formule I, 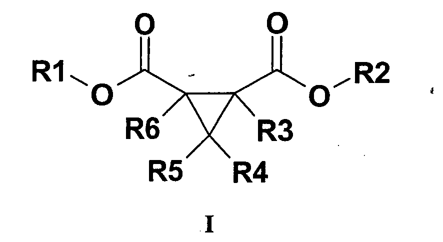 dans laquelle les significations sont
R1 H;
R2 (C₁-C₁₀)-alkyle, (C₂-C₁₀)-alcényle, (C₂-C₁₀)-alcynyle, où les radicaux alkyle, alcényle et alcynyle peuvent être substitués une ou plusieurs fois par F, Cl, Br, CF₃, NO₂, CN, COOH, COO(C₁-C₆)alkyle, (C₃-C₇)-cycloalkyle, (C₁-C₁₀)-alkyle, (C₂-C₆)-alcényle, (C₂-C₆)-alcynyle, O-(C₁-C₆)-alkyle, O-CO-(C₁-C₆)-alkyle, O-CO-(C₁-C₆)-aryle ;
R3, R4, R5, R6 indépendamment les uns des autres H, F, (C₁-C₄)-alkyle ;
et des sels physiologiquement tolérés de ceux-ci, pour la production d'un médicament destiné au traitement du syndrome métabolique.

2. Utilisation des composés de formule I selon la revendication 1 et des sels physiologiquement tolérés de ceux-ci pour la production d'un médicament destiné à réduire les taux des lipides plasmatiques.

3. Utilisation des composés de formule I selon la revendication 1 et des sels physiologiquement tolérés de ceux-ci pour la production d'un médicament destiné à réduire les taux des acides gras libres (AGL) plasmatiques.

4. Utilisation des composés de formule I selon la revendication 1 et des sels physiologiquement tolérés de ceux-ci pour la production d'un médicament destiné à réduire les taux de glycérol plasmatique.

5. Utilisation des composés de formule I selon la revendications 1 et des sels physiologiquement tolérés de ceux-ci pour la production d'un médicament destiné à réduire les taux de triglycérides plasmatiques.

6. Utilisation des composés de formule I selon la revendication 1 et des sels physiologiquement tolérés de ceux-ci pour la production d'un médicament destiné à la prophylaxie du diabète de type 2.

7. Utilisation des composés de formule I selon la revendication 1 et des sels physiologiquement tolérés de ceux-ci pour la production d'un médicament destiné au traitement de la dyslipidémie diabétique.
